# EUROPEAN PATENT APPLICATION

(11) **EP 2 586 441 A1**
(43) Date of publication of application: **01.05.2013**
(21) Application number: 11798201.7
(22) Date of filing: 23.06.2011
(51) Int. Cl.: A61K 31/353, A61K 31/53, A61K 45/00, A61P 3/04, A61P 9/10, A61P 21/00, A61P 29/00, A61P 35/00, A61P 37/08, C07D 311/62, C07D 487/04

(54) **COMBINATION OF EGCG OR METHYLATED EGCG AND A PDE INHIBITOR**

(30) Priority: 23.06.2010 JP 2010142520
(71) Applicant: Kyushu University National University Corporation, Higashi-ku Fukuoka-shi Fukuoka 812-8581 (JP)
(72) Inventor: TACHIBANA, Hirofumi, Fukuoka-shi Fukuoka 812-8581 (JP)
(74) Representative: HOFFMANN EITLE
(86) International application number: PCT/JP2011/064349
(87) International publication number: WO 2011/162320

(57) **Abstract**

Disclosed is a medicament characterized by the combination of: epigallocatechin gallate, methylated epigallocatechin gallate, or a pharmacologically permitted salt thereof; and a phosphodiesterase inhibitor.

## Description

### TECHNICAL FIELD

The present invention relates to a combination of epigallocatechin gallate (hereinafter, also referred to as "EGCG") or a methylated epigallocatechin gallate (hereinafter, also referred to as "a methylated EGCG") and a phosphodiesterase (hereinafter, also referred to as "PDE") inhibitor, which is useful for prevention or treatment of a disease, in particular, a disease such as malignant tumor, allergy, obesity, arteriosclerosis, inflammation, or muscle atrophy.

### BACKGROUND ART

Cancer is a disease accounting for one-third of the causes of death in Japan, and establishment of appropriate therapeutic approaches is strongly demanded. The therapeutic results in leukemia known as a cancer of blood cells are being improved by introducing differentiation-inducing therapy using all-trans-retinoic acid (ATRA) or use of a molecular target drug for a BCR/ABL protein produced by the Philadelphia chromosome. However, tumors acquire resistance to these drugs, and the therapy becomes difficult in many cases. Thus, there are still many problems. Accordingly, development of anti-cancer agents having action mechanisms absolutely different from those of conventional agents gives new options to patients on whom conventional anti-cancer agents are not effective. Furthermore, an anti-cancer agent having a different dose-limiting toxicity from that of conventional therapy can be used in combination with the conventional therapy to allow designing of more effective therapeutic strategy.

EGCG, one of main catechins contained in green tea, is known to have an anti-cancer activity and is at present under clinical studies for chronic lymphocytic leukemia patients (Non Patent Literatures 1 and 2). The present inventors have identified until now a 67-kDa laminin receptor (67LR) as a target molecule on cell membrane bearing the anti-cancer activity of EGCG (Non Patent Literatures 3 to 6). At first, the 67LR was discovered as a cell membrane protein binding to laminin, a main structural component of basement membrane (Non Patent Literature 7). Recent studies revealed that the expression of 67LR is abnormally enhanced in cancer cells (Non Patent Literature 8) and that there is a high correlation between the expression and proliferation, invasion, or metastasis (Non Patent Literatures 9 to 15). Also regarding lymphocytes, there are many reports that 67LR is almost not expressed in normal lymphocytes, but is highly expressed in leukemia cells (Non Patent Literatures 16 and 17). Recently, it was reported that EGCG kills selectively only leukemia cells through 67LR (Non Patent Literatures 16 and 17). This demonstrates that EGCG can be a molecular target drug for 67LR-positive leukemia cells. However, the lethal activity of EGCG on leukemia cells is restricted (Non Patent Literature 2). Enhancement of the activity is highly demanded for utilizing EGCG as an anti-cancer agent.

EGCG is further known to show activities, through 67LR, such as an anti-allergic activity (Non Patent Literatures 18 to 20), an anti-obesity activity (Non Patent Literatures 21 and 22), an anti-arteriosclerosis activity (Non Patent Literature 23), an anti-inflammatory activity (Non Patent Literature 24), and a muscle atrophy inhibitory activity (Non Patent Literature 25), and enhancement of the activities is highly demanded for clinical application of EGCG.

Furthermore, a methylated EGCG is also known to have activities such as an anti-cancer activity (Patent Literature 1) and an anti-allergic activity (Non Patent Literature 26), and also enhancement of the activities is highly demanded.

### CITATION LIST

### PATENT LITERATURE

Patent Literature 1: International Publication No. WO2009/107262

### NON PATENT LITERATURE

Non Patent Literature 1: Cancer res., 2006, 66:2500-2505
Non Patent Literature 2: J. Clin. Oncol., 2009, 27:3808-3814
Non Patent Literature 3: Nat. Struct. Mol. Biol., 2004, 11:380-381
Non Patent Literature 4: Biochem. Biophys. Res. Commun., 2005, 333:628-635
Non Patent Literature 5: Biochem. Biophys. Res. Commun., 2008, 371:172-176
Non Patent Literature 6: J. Biol. Chem., 2008, 283:3050-3058
Non Patent Literature 7: Biosci. Rep., 2008, 28:33-48
Non Patent Literature 8: J. Natl. Cancer Inst., 1991, 83:29-36
Non Patent Literature 9: J. Cell. Biochem., 1997, 67:155-165
Non Patent Literature 10: Breast Cancer Res Treat., 1998, 52:137-145
Non Patent Literature 11: Cancer, 1993, 72:455-461
Non Patent Literature 12: J. Pathol., 1996, 179:376-380
Non Patent Literature 13: Brain Res. Bull., 2009, 79:402-408
Non Patent Literature 14: Clin. Cancer Res., 1997, 3:227-231
Non Patent Literature 15: Clin. Cancer Res., 1996, 2:1777-1780
Non Patent Literature 16: Br. J. Haematol., 2010, 149:55-64
Non Patent Literature 17: Blood, 2006, 108:2804-2810
Non Patent Literature 18: Biochem. Biophys. Res. Commun., 2005, 336:674-681
Non Patent Literature 19: Biochem. Biophys. Res. Commun., 2006, 348: 524-531
Non Patent Literature 20: Arch. Biochem. Biophys., 2008, 76: 133-138
Non Patent Literature 21: Am. J. Physiol. Cell. Physiol., 2009, 297:C121-132
Non Patent Literature 22: Mol. Nutr. Food Res., 2009, 53:349-360
Non Patent Literature 23: J. Mol. Cell. Cardiol., 2010, 48:1138-1145
Non Patent Literature 24: J. Immunol., 2010, 185:33-45
Non Patent Literature 25: Biofactors, 2009, 35:279-29
Non Patent Literature 26: Biochem. Biophys. Res. Commun., 2007, 364:79-85

### SUMMARY OF INVENTION

The present inventors have recently found that NO production through 67LR is important in lethal activity of EGCG on a multiple myeloma cell line (Reference 18 shown below). Accordingly, the inventors have expected that the anti-cancer activity of EGCG may be enhanced by promoting an NO-dependent signaling pathway. Cyclic GMP is a mediator playing an important role in NO signaling (Reference 19 shown below), and inhibition of a cGMP-degrading enzyme, phosphodiesterase, is known to promote NO signaling.

Phosphodiesterase is an enzyme involved in degradation of second messengers, cGMP and cAMP, important in vivo and is attracting attention as a drug discovery target for cardiovascular diseases (Reference 20 shown below) and male sexual dysfunction, erectile dysfunction (References 21 and 22 shown below). It has been also revealed that phosphodiesterase 5 (PDE5) is highly expressed in tumor cells (Reference 23 shown below).

Accordingly, the present inventors have arrived at the idea that the anti-cancer activity of EGCG may be enhanced by using it in combination with a PDE inhibitor.

The inventors have found that the cancer cell-killing activity of EGCG is enhanced by a combination of a PDE inhibitor and EGCG, and have arrived at the present invention.

Though a large number of studies of enhancing the anti-cancer activity of EGCG have been reported until now, no studies focused on combination of EGCG with a PDE inhibitor. The present invention has revealed that the combined use notably enhances the anti-cancer activity.

Since 67LR participates in the anti-cancer activity of EGCG, it is believed that a combination of EGCG and a PDE inhibitor is effective not only for cancers but also for diseases on which EGCG is known to exert an activity through 67LR.

The gist of the present invention is as follows:
[1] A pharmaceutical composition comprising a combination of epigallocatechin gallate or a methylated epigallocatechin gallate or a pharmaceutically acceptable salt thereof and a phosphodiesterase inhibitor;
[2] The composition according to aspect [1], which composition is used in treatment of cancer, allergy, obesity, arteriosclerosis, inflammation, or muscle atrophy;
[3] The composition according to aspect [1] or [2], which composition is used in treatment of a 67LR-positive disease;
[4] The composition according to any one of aspects [1] to [3], wherein the phosphodiesterase inhibitor is a phosphodiesterase 3 inhibitor or phosphodiesterase 5 inhibitor;
[5] The composition according to any one of aspects [1] to [3], wherein the phosphodiesterase inhibitor is vardenafil;
[6] A therapeutic method for cancer, allergy, obesity, arteriosclerosis, inflammation, or muscle atrophy, the method comprising administering a combination of epigallocatechin gallate or a methylated epigallocatechin gallate or a pharmaceutically acceptable salt thereof and a phosphodiesterase inhibitor to a patient; and
[7] A kit for use in treatment of cancer, allergy, obesity, arteriosclerosis, inflammation, or muscle atrophy, the kit comprising:
   a) a first unit dosage form containing epigallocatechin gallate, a methylated epigallocatechin gallate, or a pharmaceutically acceptable salt thereof;
   b) a second unit dosage form containing a phosphodiesterase inhibitor; and
   c) a container device for containing the first unit dosage form and the second unit dosage form.

### BRIEF DESCRIPTION OF DRAWINGS

Figure 1 shows the effects of a combination of EGCG and a PDE5 inhibitor in the cell-killing activity (A) and the cytostatic activity (B) of EGCG on multiple myeloma cells.
Figure 2 shows the effects of a combination of EGCG and a PDE5 inhibitor in the cell-killing activity (A) and the cytostatic activity (B) of EGCG on acute myeloid leukemia cells.
Figure 3 shows the influences of 67LR in the cell-killing activities (A) and the cytostatic activities (B) of EGCG and a PDE5 inhibitor on multiple myeloma cells.
Figure 4 shows the influences of 67LR in the cell-killing activities (A) and the cytostatic activities (B) of EGCG and a PDE5 inhibitor on acute myeloid leukemia cells.
Figure 5 shows the influences of 67LR in the apoptosis-inducing activities of EGCG and a PDE5 inhibitor on multiple myeloma cells.
Figure 6 shows the influences of 67LR in the apoptosis-inducing activities of EGCG and a PDE5 inhibitor on acute myeloid leukemia cells.
Figure 7 shows the effects of a combination of EGCG and a PDE5 inhibitor in the cell-killing activity (A) and the cytostatic activity (B) of EGCG on a human prostate cancer cell line, PC3.
Figure 8 shows the effects of a combination of EGCG and a PDE5 inhibitor in the cell-killing activity and the cytostatic activity of EGCG on a human multiple myeloma cell line, RPMI8226.
Figure 9 shows the effects of a combination of EGCG and a PDE5 inhibitor in the cell-killing activity and the cytostatic activity of EGCG on multiple myeloma derived from a patient.
Figure 10 shows the effects of a combination of EGCG and a PDE5 inhibitor in the cell-killing activity and the cytostatic activity of EGCG on a human plasmacytoma, ARH77.
Figure 11 shows the effects of a combination of EGCG and a PDE5 inhibitor in the cell-killing activity and the cytostatic activity of EGCG on a chronic myeloid leukemia cell line, K562.
Figure 12 shows the effects of a combination of EGCG and a PDE5 inhibitor in the cytostatic activity of EGCG on a human breast cancer cell line, MCF-7.
Figure 13 shows the effects of a combination of EGCG and a PDE5 inhibitor in the cytostatic activity of EGCG on a human gastric cancer cell line, MKN45.
Figure 14 shows the effects of a combination of EGCG and a PDE5 inhibitor in the cell-killing activity and the cytostatic activity of EGCG on a human pancreatic cancer cell line, PANC-1.
Figure 15 shows the effects of a combination of EGCG and a PDE5 inhibitor on the viability and cell proliferation of a human umbilical vein endothelial cell, HUVEC.
Figure 16 shows the effects of a combination of EGCG and a PDE5 inhibitor on the viability and cell proliferation of peripheral blood lymphocytes derived from a healthy subject.
Figure 17 shows the effects of a combination of EGCG and a PDE5 inhibitor on tumor formation of a mouse plasmacytoma cell line, MPC11, transplanted to Balb/c mice.
Figure 18 shows the effects of a combination of a methylated EGCG and a PDE5 inhibitor on a human multiple myeloma cell line, U266.
Figure 19 shows the effects of a combination of a methylated EGCG and a PDE5 inhibitor on a prostate cancer cell line, PC-3, and a pancreatic cancer cell line, PANC-1.
Figure 20 shows the effects of a combination of EGCG and a PDE3 inhibitor in the cytostatic activity of EGCG on a chronic myeloid leukemia cell line, K562.

### DESCRIPTION OF EMBODIMENTS

The present invention provides an agent suitable for the use in treatment of a disease, the agent comprising a combination of epigallocatechin gallate or its pharmaceutically acceptable salt or a methylated epigallocatechin gallate or its pharmaceutically acceptable salt and a phosphodiesterase inhibitor. The invention also provides a therapeutic method of a disease, the method comprising administering a combination of EGCG or its pharmaceutically acceptable salt or a methylated EGCG or its pharmaceutically acceptable salt and a PDE inhibitor to a patient. The invention is useful for treatment of, in particular, diseases such as cancers, allergy, obesity, arteriosclerosis, inflammation, and muscle atrophy or 67LR-positive diseases.

Throughout the specification, the term "combination" refers to a combination in which the respective components of the combination are administered to a patient simultaneously, continuously, or separately. Therefore, the agent including a combination of EGCG or a methylated EGCG or a pharmaceutically acceptable salt thereof and a PDE inhibitor according to the present invention may be an agent in a form where the two components are contained in a single unit dosage form or an agent in a form where a first unit dosage form containing EGCG or a methylated EGCG or a pharmaceutically acceptable salt thereof and a second unit dosage form containing a PDE inhibitor are combined.

Throughout the specification, the term "67LR-positive disease" refers to a disease in which the expression of 67LR is abnormally enhanced in the site (cells) of the disease, such as leukemia cells highly expressing 67LR.

EGCG ((-)-epigallocatechin-3-O-gallate) is one of polyphenol compounds contained in, for example, green tea leaves.

The methylated EGCG is a compound represented by Formula (I): 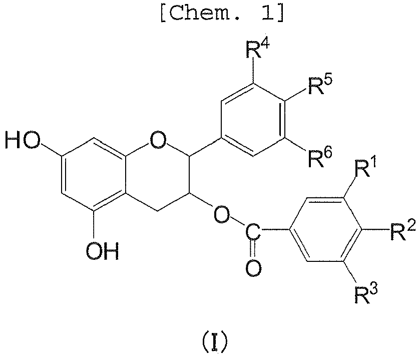 (wherein R¹ to R⁶ each independently represent a hydrogen atom, a hydroxyl group, or a methoxy group, and at least one of R¹ to R⁶ represents a methoxy group). Examples of the compound include compounds where one of R¹ to R⁶ is a methoxy group, and the others are hydroxyl groups; and compounds where two of R¹ to R⁶ are methoxy groups, and the others are hydroxyl groups. Specific examples of the compound include 3"-O-methyl-epigallocatechin-3-gallate, 4"-O-methyl-epigallocatechin-3-gallate, 3",4"-O-dimethyl-epigallocatechin-3-gallate, 3",5"-O-dimethyl-epigallocatechin-3-gallate, 4'-O-methyl-epigallocatechin-3-gallate, 4',4"-O-dimethyl-epigallocatechin-3-gallate, 4',3",4"-O-trimethyl-epigallocatechin-3-gallate, 4',3",5"-O-trimethyl-epigallocatechin-3-gallate, 3',5'-O-dimethyl-epigallocatechin-3-gallate, 3',5',4"-O-trimethyl-epigallocatechin-3-gallate, 3',5',3",4"-O-tetramethyl-epigallocatechin-3-gallate, 3',5',3",5"-O-tetramethyl-epigallocatechin-3-gallate, 3',4',5'-O-trimethyl-epigallocatechin-3-gallate, 3',4',5',4"-O-tetramethyl-epigallocatechin-3-gallate, 3',4',5',3",4"-O-pentamethyl-epigallocatechin-3-gallate, and 3',4',5',3",5"-O-pentamethyl-epigallocatechin-3-gallate. The methylated EGCG shows various activities through 67LR, like EGCG. Preferable examples of the methylated EGCG are 3"-O-methyl-epigallocatechin-3-gallate and 4"-O-methyl-epigallocatechin-3-gallate.

Examples of the methylated EGCG include compounds represented by Formula (II):

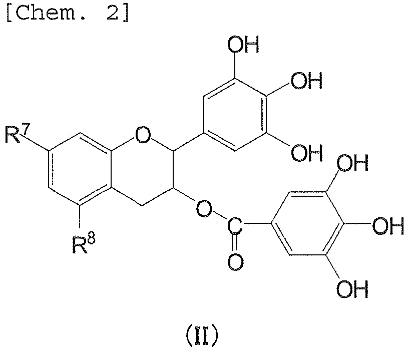

(wherein, R⁷ and R⁸ each independently represent a hydroxyl group or a methoxy group, and at least one of R⁷ and R⁸ represents a methoxy group). A specific example of the compound is 7-O-methyl-epigallocatechin-3-gallate.

EGCG and the methylated EGCG can be isolated from, for example, extract of green tea leaves by a method such as HPLC, synthetic adsorbent chromatography, ion-exchange chromatography, or gel filtration. Alternatively, EGCG and the methylated EGCG can be synthesized by a known method.

In the present invention, EGCG and the methylated EGCG can also be used as pharmaceutically acceptable salts thereof. Examples of the pharmaceutically acceptable salt include salts with alkali metals (e.g., sodium, potassium, and lithium) and salts with alkaline earth metals (e.g., calcium and magnesium). The salt can be produced by, for example, reacting EGCG or a methylated EGCG with a hydroxide or hydride of an alkali metal or alkaline earth metal.

The phosphodiesterase inhibitors are materials that inhibit enzymes hydrolyzing cyclic phosphodiesters such as cAMP and cGMP, and non-selective PDE inhibitors and selective PDE inhibitors are known. In the present invention, both the non-selective PDE inhibitors and the selective PDE inhibitors can be used.

Examples of the non-selective PDE inhibitor include caffeine, theophylline, theobromine, 3-isobutyl-1-methylxanthine (IBMX), and pentoxifylline.

Examples of the selective PDE inhibitor include PDE1 inhibitors such as 8-methoxy-3-isobutyl-1-methylxanthine; PDE2 inhibitors such as erythro-9-(2-hydroxy-3-nonyl)adenine hydrochloride (EHNA hydrochloride); PDE3 inhibitors such as cilostamide, milrinone, and trequisin; PDE4 inhibitors such as etazolate hydrochloride and denbufylline; and PDE5 inhibitors such as vardenafil, sildenafil, zaprinast, tadalafil, dipyridamole, 4-{[3',4'-(methylenedioxy)benzyl]amino}-6-methoxyquinazoline, and 1-(3-chloroanilino)-4-phenylphthalazine (MY-5445). In the present invention, PDE3 inhibitors and PDE5 inhibitors are preferable, and a combination of EGCG or its pharmaceutically acceptable salt and a PDE5 inhibitor and a combination of EGCG or its pharmaceutically acceptable salt and a PDE3 inhibitor are preferable.

The present invention is characterized by combining EGCG or its pharmaceutically acceptable salt or a methylated EGCG or its pharmaceutically acceptable salt and a PDE inhibitor. As understood by the following Examples, the use of these two components in combination shows a notably enhanced lethal activity on cancer cells compared to the cases of using each of them alone. In addition, it was revealed that the anti-cancer activity by the combined use of EGCG and a PDE5 inhibitor is completely dependent on 67LR and that the anti-cancer activity selectively acts on 67LR-positive cancer cells without causing any significant side effect to 67LR-negative normal cells.

Accordingly, the agent including a combination of EGCG or its pharmaceutically acceptable salt or a methylated EGCG or its pharmaceutically acceptable salt and a PDE inhibitor of the present invention is useful for cancer therapy. The agent of the present invention is particularly useful for treatment of cancers (e.g., multiple myeloma, acute myeloid leukemia, prostate cancer, melanoma, breast cancer, lung cancer, cervical cancer, and colon cancer) in which EGCG shows the anti-cancer activity through 67LR.

Furthermore, the present invention is based on that the activity of EGCG or a methylated EGCG is enhanced by using as a combination with a PDE inhibitor. Consequently, the agent of the present invention is useful for treatment of not only cancers but also of 67LR-positive diseases, diseases of which therapy by each of EGCG and the methylated EGCG alone is effective, and diseases of which therapy by EGCG or the methylated EGCG through 67LR is effective, such as allergy, obesity, arteriosclerosis, inflammation, and muscle atrophy.

The agent of the present invention can be provided in a dosage form suitable for administration prepared by applying a pharmaceutically acceptable diluent or carrier to the active ingredient (EGCG, a methylated EGCG, or a pharmaceutically acceptable salt thereof, or a PDE inhibitor). Any diluent or carrier that is pharmaceutically acceptable can be used without limitation, and examples thereof include a binder, a disintegrator, an acidifier, a foaming agent, an artificial sweetener, a flavoring, a lubricant, a coloring agent, a stabilizer, a buffer, an antioxidant, and a surfactant. The dosage form is not particularly limited, and the agent can be provided in a form of, for example, solid preparations such as powder, granules, capsules, pills, and tablets; and liquid preparations such as liquid agents, suspensions, and emulsions. These preparations can be produced by a common method using a diluent or carrier.

The dose of EGCG, a methylated EGCG, or a pharmaceutically acceptable salt thereof and the dose of a PDE inhibitor are effective doses for exerting a desired therapeutic effect on a target disease, for example, effective doses for providing a desired anti-cancer activity.

The dose per day of EGCG, a methylated EGCG, or a pharmaceutically acceptable salt thereof is, for example, 0.01 mg to 5000 mg, 0.1 mg to 50 mg, 0.5 mg to 20 mg, or 1.0 mg to 10 mg.

The dose per day of a PDE inhibitor is, for example, 0.01 mg to 2000 mg, 0.1 mg to 50 mg, 0.5 mg to 20 mg, or 1.0 mg to 10 mg.

Alternatively, the dose per day of a PDE inhibitor may be, for example, 0.01 to 100 parts by mass, 0.1 to 10 parts by mass, or 0.5 to 5 parts by mass based on 1 part by mass of the dose per day of EGCG, a methylated EGCG, or a pharmaceutically acceptable salt thereof.

The agent including a combination of EGCG, a methylated EGCG, or a pharmaceutically acceptable salt thereof and a PDE inhibitor according to the present invention may be used alone for treating cancer or may be used in combination with another anti-cancer agent or may be used in combination with surgery or radiation therapy.

### EXAMPLES

The present invention will now be described more specifically by Examples and Comparative Examples, but is not limited to these Examples.

### [Materials and methods]

### (1) Investigation of cytostatic activity and cell-killing activity of combination of EGCG and PDE5 inhibitor

A human multiple myeloma cell line, U266, was subcultured in an RPMI 1640 medium (NISSUI PHARMACEUTICAL Co. LTD., Tokyo) supplemented with 10% fetal calf serum (FCS) (BIOLOGICAL INDUSTRIES) at 37°C under vapor-saturated 5% CO₂ conditions. The cells were cultured in the logarithmic growth phase. A human acute myeloid leukemia cell line, HL60, was subcultured in an RPMI 1640 medium supplemented with 10% fetal calf serum (FCS) at 37°C under vapor-saturated 5% CO2 conditions. The cells were cultured in the logarithmic growth phase. The RPMI 1640 medium used in the culture was prepared by suspending 10.4 g of an RPMI 1640 medium (Cosmo Bio Co., Ltd., Tokyo), 2.38 g of HEPES (Wako Pure Chemical Industries, Ltd., Osaka), 0.5 vial of Penicillin G Potassium for Injection 200000 Unit (Meiji Seika Kaisha, Limited, Tokyo), 0.1 vial of Streptomycin Sulfate for Injection 1 g (Meiji Seika Kaisha, Limited, Tokyo), and 2.0 g of NaHCO3 (Nacalai tesque, Inc., Kyoto) in 1 L of ultrapure water, followed by filter sterilization. Subsequently, the RPMI 1640 medium was supplemented with fetal calf serum (FCS) and was used in cell culture.

A trypan blue staining solution was prepared by suspending trypan blue (Wako Pure Chemical Industries, Ltd.) in PBS at a concentration of 1% and was used in investigation of the anti-cancer activity of a combination of EGCG and vardenafil by trypan blue assay. The PBS used was prepared by dissolving 8.0 g of NaCl (Nacalai tesque, Inc., Kyoto), 0.2 g of KCl (Nacalai tesque, Inc., Kyoto), 1.15 g of Na₂HPO₄ (Nacalai tesque, Inc., Kyoto), and 0.2 g of KH₂PO (Nacalai tesque, Inc., Kyoto) in 1 L of ultrapure water, followed by autoclave sterilization.

Cell lines, U266 and HL60, adjusted to be 5×10⁴ cells/mL (U266) and 1×10⁴ cells/mL (HL60), respectively, in an RPMI 1640 medium supplemented with 1% FCS were seeded in a 24-well plate (Falcon Co., Ltd.). Vardenafil was added thereto at a final concentration of 5 µM. The vardenafil (Toronto Research Chemicals Inc.) used was adjusted to be 5 mM with ultrapure water containing 10% dimethyl sulfoxide (DMSO) (Nacalai tesque, Inc., Kyoto) and was stored at -30°C. It was thawed at any time before use.

The plate was returned to the incubator immediately after the addition of vardenafil. After 3 hours, EGCG (Sigma Corporation) was added thereto at a final concentration of 5 µM. The EGCG used was adjusted to be 5 mM with ultrapure water containing 10% dimethyl sulfoxide (DMSO) (Nacalai tesque, Inc., Kyoto) and was stored at -30°C. It was thawed at any time before use.

The plate was returned to the incubator immediately after the addition of EGCG, followed by culturing for 96 hours. Subsequently, a trypan blue staining solution was added to the sufficiently suspended cell culture solution at a final concentration of 0.1%. After 10 minutes, the number of the cells was counted with a hemocytometer (Becton, Dickinson and Company). Unstained cells were deemed as viable cells, and the cell viability was determined as a ratio of the number of unstained cells in trypan blue staining to the total number of cells. Subsequently, the cells were photographed with a microscope (Keyence Corporation). The statistical treatment of the experimental results was performed by a Tukey's test.

### (2) Investigation of involvement of 67LR in cytostatic activity and cell-killing activity of combination of EGCG and PDE5 inhibitor

A trypan blue staining solution was prepared by suspending trypan blue in PBS at a concentration of 1% and was used in investigation of the anti-cancer activity of a combination of EGCG and vardenafil by trypan blue assay. The PBS used was prepared by dissolving 8.0 g of NaCl, 0.2 g of KCl, 1.15 g of Na₂HPO₄, and 0.2 g of KH₂PO₄ in 1 L of ultrapure water, followed by autoclave sterilization.

A human multiple myeloma cell line, U266, and a human acute myeloid leukemia cell line, HL60, adjusted to be 5×10⁴ cells/mL (U266) and 1×10⁴ cells/mL (HL60), respectively, in an RPMI 1640 medium supplemented with 1% FCS were seeded in a 96-well plate (Falcon Co., Ltd.). Vardenafil was added thereto at a final concentration of 5 µM. Immediately after the addition, an anti-67LR monoclonal antibody, MLuc5 (Abcam PLC), was added thereto at a final concentration of 20 µg/mL. In the group of adding an isotype control antibody, mouse IgM (Cosmo Bio Co., Ltd., Tokyo) was added at a final concentration of 20 µg/mL. The vardenafil used was adjusted to be 5 mM with ultrapure water containing 10% dimethyl sulfoxide (DMSO) and was stored at -30°C. It was thawed at any time before use.

The plate was returned to the incubator immediately after the addition of each antibody. After 3 hours, EGCG was added thereto at a final concentration of 5 µM. The EGCG used was adjusted to be 5 mM with ultrapure water containing 10% dimethyl sulfoxide (DMSO) and was stored at -30°C. It was thawed at any time before use.

The plate was returned to the incubator immediately after the addition of EGCG, followed by culturing for 96 hours. Subsequently, a trypan blue staining solution was added to the sufficiently suspended cell culture solution at a final concentration of 0.1%. After 10 minutes, the number of the cells was counted with a hemocytometer. Unstained cells were deemed as viable cells, and the cell viability was determined as a ratio of the number of unstained cells in trypan blue staining to the total number of cells. The statistical treatment of the experimental results was performed by a Tukey's test.

### (3) Investigation of involvement of 67LR in apoptosis-inducing activity of combination of EGCG and PDE5 inhibitor (vardenafil)

A propidium iodide (PI) staining solution was prepared by suspending PI (Sigma Corporation) in PBS at a concentration of 50 µg/mL and was used in investigation of the anti-cancer activity of a combination of EGCG and vardenafil by Annexin V PI double staining. The PBS used was prepared by dissolving 8.0 g of NaCl, 0.2 g of KCl, 1.15 g of Na₂HPO₄, and 0.2 g of KH₂PO₄ in 1 L of ultrapure water, followed by autoclave sterilization. The Annexin V Binding Buffer used was prepared with 10 mM HEPES, 140 mM NaCl, and 2.5 mM CaCl₂ (Wako Pure Chemical Industries, Ltd.) to be pH 7.4, followed by filter sterilization.

Cell lines, U266 and HL60, adjusted to be 5×10⁴ cells/mL (U266) and 1×10⁴ cells/mL (HL60), respectively, in an RPMI 1640 medium supplemented with 1% FCS were seeded in a 96-well plate. Vardenafil was added thereto at a final concentration of 5 µM. Immediately after the addition, an anti-67LR monoclonal antibody, MLuc5, was added thereto at a final concentration of 20 µg/mL. In the group of adding an isotype control antibody, mouse IgM was added at a final concentration of 20 µg/mL.

The vardenafil used was adjusted to be 5 mM with ultrapure water containing 10% dimethyl sulfoxide (DMSO) and was stored at -30°C. It was thawed at any time before use. The plate was returned to the incubator immediately after the addition of the respective antibodies. After 3 hours, EGCG was added thereto at a final concentration of 5 µM.
The EGCG used was adjusted to be 5 mM with ultrapure water containing 10% dimethyl sulfoxide (DMSO) and was stored at - 30°C. It was thawed at any time before use.

The plate was returned to the incubator immediately after the addition of EGCG, followed by culturing for 96 hours. Subsequently, the sufficiently suspended cell culture solution was centrifuged at 300xg for 5 minutes. The supernatant was removed, and the cells were suspended in an Annexin V Binding uffer, and the number of the cells was counted with a hemocytometer. The suspension was centrifuged again at 300×g for 5 minutes, and the cells were suspended in an Annexin V Binding Buffer at a concentration of 1×10⁶ cells/mL. Five micrograms of an Annexin V Alexa Fluor 488 Conjugate (Invitrogen Corporation) was added to 100 µL of the cell suspension, and 2 µL of a PI staining solution prepared in advance was further added thereto from above, followed by leaving to stand at room temperature for 15 minutes. Subsequently, 400 µL of an Annexin V Binding Buffer was added thereto from above, and the cells were counted with a flow cytometer, FACS Caliber (Becton, Dickinson and Company). After compensation, the Annexin V Alexa Fluor 488 Conjugate was measured at FL 1, and the cells deemed to be positive were determined as apoptotic cells. The analysis was performed with Cell Quest (Becton, Dickinson and Company). The statistical treatment of the experimental results was performed by a Tukey's test.

### (4) Investigation of cytostatic activity and cell-killing activity of combination of EGCG and PDE5 inhibitor on human prostate cancer

A human prostate cancer cell line, PC3, was subcultured in an RPMI 1640 medium supplemented with 10% fetal calf serum (FCS) at 37°C under vapor-saturated 5% CO₂ conditions. The cells were cultured in the logarithmic growth phase. The RPMI 1640 medium used in the culture was prepared by suspending 10.4 g of an RPMI 1640 medium, 2.38 g of HEPES, 0.5 vial of Penicillin G Potassium for Injection 200000 Unit, 0.1 vial of Streptomycin Sulfate for Injection 1 g, and 2.0 g of NaHCO₃ in 1 L of ultrapure water, followed by filter sterilization. Subsequently, the RPMI 1640 medium was supplemented with fetal calf serum (FCS) and was used in cell culture.

A trypan blue staining solution was prepared by suspending trypan blue in PBS at a concentration of 1% and was used in investigation of the anti-cancer activity of a combination of EGCG and vardenafil by trypan blue assay. The PBS used was prepared by dissolving 8.0 g of NaCl, 0.2 g of KCl, 1.15 g of Na₂HPO₄, and 0.2 g of KH₂PO₄ in 1 L of ultrapure water, followed by autoclave sterilization.

The cell line, PC3, adjusted to be 1×10⁴ cells/mL in an RPMI 1640 medium supplemented with 10% FCS was seeded in a 24-well plate (NUNC Inc.). After pre-culturing for 24 hours, the medium was replaced with an RPMI medium supplemented with 2% FCS and containing vardenafil so as to give a final concentration of 5 µM. The vardenafil used was adjusted to be 5 mM with ultrapure water containing 10% dimethyl sulfoxide (DMSO) and was stored at -30°C. It was thawed at any time before use. The plate was returned to the incubator immediately after the addition of vardenafil. After 2 hours, EGCG was added thereto at a final concentration of 10 µM. The EGCG used was adjusted to be 5 mM with ultrapure water containing 10% dimethyl sulfoxide (DMSO) and was stored at -30°C. It was thawed at any time before use.

The plate was returned to the incubator immediately after the addition of EGCG, followed by culturing for 72 hours. Subsequently, a trypan blue staining solution was added to the sufficiently suspended cell culture solution at a final concentration of 0.1%. After 10 minutes, the number of the cells was counted with a hemocytometer. Unstained cells were deemed as viable cells, and the cell viability was determined as a ratio of the number of unstained cells in trypan blue staining to the total number of cells. The cells were photographed with a microscope. The statistical treatment of the experimental results was performed by a Tukey's test.

### [Results]

### (1) Effect of combination of EGCG and PDE5 inhibitor in cell-killing activity (A) and cytostatic activity (B) of EGCG on multiple myeloma cells

Enhancement of the activity of EGCG on multiple myeloma by a selective inhibitor (vardenafil) for PDE5, which is an enzyme specifically degrading cGMP and is believed to be an EGCG resistant factor, was investigated.

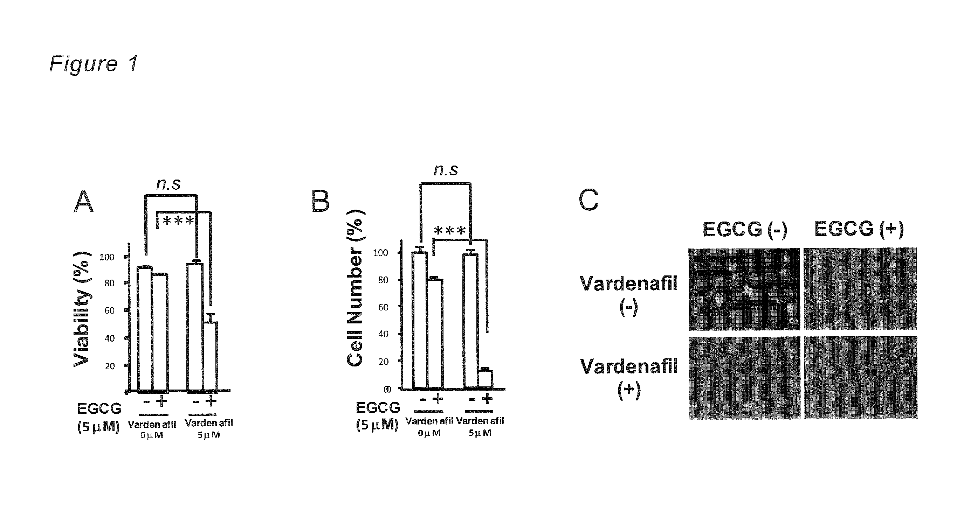

A human multiple myeloma cell line, U266, was seeded in a 24-well plate at 5×10⁴ cells/mL, and vardenafil was added thereto at a final concentration of 5 µM. After 3 hours, EGCG was added thereto at a final concentration of 5 µM. After 96 hours, cell viability determination and viable cell count were performed by trypan blue staining using a hemocytometer. The results are shown in Figure 1 (n=3, Tukey's test, ***: P<0.001).

As shown in Figure 1, though any cytotoxicity was not observed with EGCG and vardenafil alone, a combination of EGCG and vardenafil significantly strongly decreased the viability of cancer cells: (A). The activity of EGCG on viable cell number was notably enhanced by the presence of vardenafil: (B). The same results were obtained also in the microscopic photograph: (C). These results demonstrate that vardenafil, a PDE5-selective inhibitor, notably enhances the activity of EGCG on multiple myeloma cells.

### (2) Effect of combination of EGCG and PDE5 inhibitor in cell-killing activity (A) and cytostatic activity (B) of EGCG on acute myeloid leukemia cells

Enhancement of the activity of EGCG on acute myeloid leukemia cells by a selective inhibitor (vardenafil) for PDE5, which is an enzyme specifically degrading cGMP and is believed to be an EGCG resistant factor, was investigated.

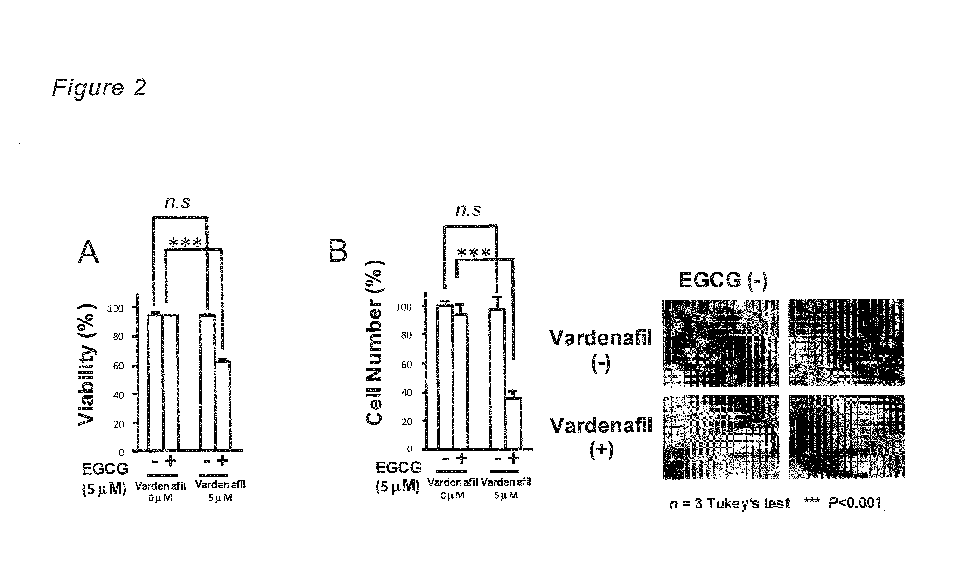

A human acute myeloid leukemia cell, HL60, was seeded in a 24-well plate at 1×10⁴ cells/mL, and vardenafil was added thereto at a final concentration of 5 µM. After 3 hours, EGCG was added thereto at a final concentration of 5 µM. After 96 hours, cell viability determination and viable cell count were performed by trypan blue staining using a hemocytometer. The results are shown in Figure 2 (n=3, Tukey's test, ***: P<0.001).

As shown in Figure 2, though any cytotoxicity was not observed with EGCG and vardenafil alone, a combination of EGCG and vardenafil significantly strongly decreased the viability of cancer cells: (A). The activity of EGCG on viable cell number was notably enhanced by the presence of vardenafil: (B). The same results were obtained also in the microscopic photograph: (C). These results demonstrate that vardenafil, a PDE5-selective inhibitor, notably enhances the activity of EGCG on acute myeloid leukemia cells.

### (3) Investigation of involvement of 67LR in cell-killing activity (A) and cytostatic activity (B) of EGCG and PDE5 inhibitor on multiple myeloma cells

Enhancement of the activity of EGCG on multiple myeloma cells by a selective inhibitor (vardenafil) for PDE5, which is an enzyme specifically degrading cGMP and is believed to be an EGCG resistant factor, was investigated.

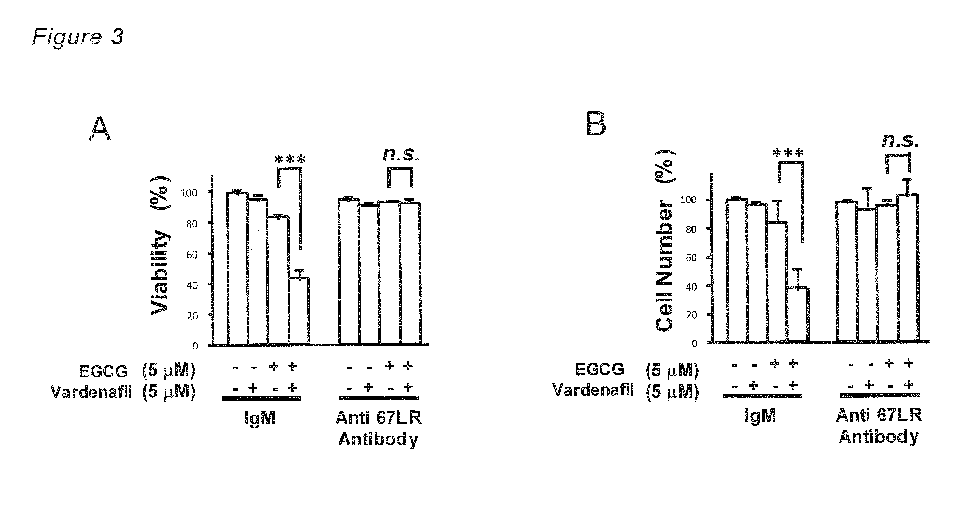

A human multiple myeloma cell line, U266, was seeded in a 96-well plate at 5×10⁴ cells/mL, and vardenafil was added thereto at a final concentration of 5 µM. Immediately after the addition, an anti-67LR monoclonal antibody, MLuc5, was added thereto at a final concentration of 20 µg/mL. In the group of adding an isotype control antibody, mouse IgM was added at a final concentration of 20 µg/mL. After 3 hours, EGCG was added thereto at a final concentration of 5 µM. After 96 hours, cell viability determination and viable cell count were performed by trypan blue staining using a hemocytometer. The results are shown in Figure 3 (n=3, Tukey's test, ***: P<0.001).

As shown in Figure 3, though any cytotoxicity was not observed with EGCG and vardenafil alone, a combination of EGCG and vardenafil significantly strongly decreased the viability of cancer cells: (A). The activity of EGCG on viable cell number was notably enhanced by the presence of vardenafil: (B). Both activities by the combination of EGCG and vardenafil were completely cancelled by the treatment with the anti-67LR antibody: (A) and (B). These results demonstrate that the cancer cell-killing activity by the combination of EGCG and vardenafil is 67LR-dependent.

### (4) Investigation of involvement of 67LR in cell-killing activity (A) and cytostatic activity (B) of EGCG and PDE5 inhibitor on acute myeloid leukemia cells

Enhancement of the activity of EGCG on acute myeloid leukemia cells by a selective inhibitor (vardenafil) for PDE5, which is an enzyme specifically degrading cGMP and is believed to be an EGCG resistant factor, was investigated.

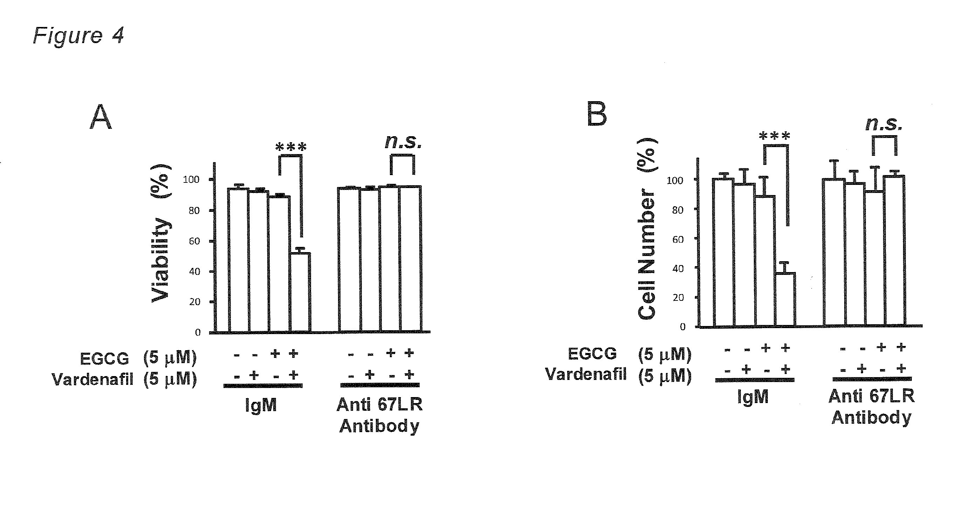

An acute myeloid leukemia cell line, HL60, was seeded in a 96-well plate at 1×10⁴ cells/mL, and vardenafil was added thereto at a final concentration of 5 µM. Immediately after the addition, an anti-67LR monoclonal antibody, MLuc5, was added thereto at a final concentration of 20 µg/mL. In the group of adding an isotype control antibody, mouse IgM was added at a final concentration of 20 µg/mL. After 3 hours, EGCG was added thereto at a final concentration of 5 µM. After 96 hours, cell viability determination and viable cell count were performed by trypan blue staining using a hemocytometer. The results are shown in Figure 4 (n=3, Tukey's test, ***: P<0.001).

As shown in Figure 4, though any cytotoxicity was not observed with EGCG and vardenafil alone, a combination of EGCG and vardenafil significantly strongly decreased the viability of cancer cells: (A). The activity of EGCG on viable cell number was notably enhanced by the presence of vardenafil: (B). Both activities by the combination of EGCG and vardenafil were completely cancelled by the treatment with the anti-67LR antibody: (A) and (B). These results demonstrate that the cancer cell-killing activity by the combination of EGCG and vardenafil is 67LR-dependent.

### (5) Investigation of involvement of 67LR in apoptosis-inducing activity of EGCG and PDE5 inhibitor on multiple myeloma cells

Enhancement of the activity of EGCG on multiple myeloma cells by a selective inhibitor (vardenafil) for PDE5, which is an enzyme specifically degrading cGMP and is believed to be an EGCG resistant factor, was investigated.

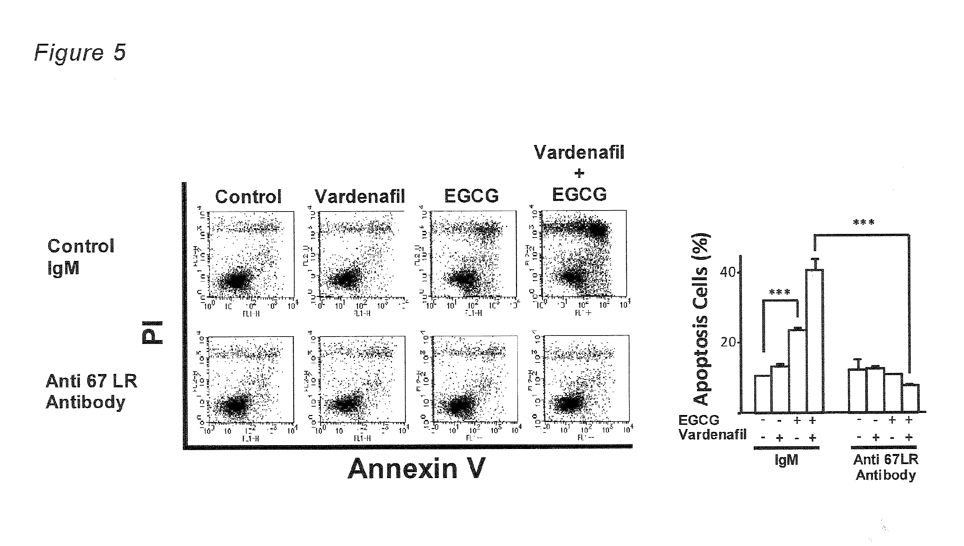

A human multiple myeloma cell line, U266, was seeded in a 96-well plate at 5×10⁴ cells/mL, and vardenafil was added thereto at a final concentration of 5 µM. Immediately after the addition, an anti-67LR monoclonal antibody, MLuc5, was added thereto at a final concentration of 20 µg/mL. In the group of adding an isotype control antibody, mouse IgM was added at a final concentration of 20 µg/mL. After 3 hours, EGCG was added thereto at a final concentration of 5 µM. After 96 hours, apoptotic cell ratio was measured by Annexin V PI double staining. The results are shown in Figure 5 (n=3, Tukey's test, ***: P<0.001).

As shown in Figure 5, any cytotoxicity was not observed with vardenafil alone, whereas apoptosis was induced by EGCG alone. Furthermore, the apoptosis-inducing activity of EGCG was notably enhanced by its combined use with vardenafil. The apoptosis-inducing activities by EGCG alone and by the combination of EGCG and vardenafil were completely cancelled by the treatment with the anti-67LR antibody. These results demonstrate that vardenafil notably enhances the 67LR-dependent apoptosis-inducing activity of EGCG on cancer cells.

### (6) Investigation of involvement of 67LR in apoptosis-inducing activity of EGCG and PDE5 inhibitor on acute myeloid leukemia cells

Enhancement of the activity of EGCG on acute myeloid leukemia cells by a selective inhibitor (vardenafil) for PDE5, which is an enzyme specifically degrading cGMP and is believed to be an EGCG resistant factor, was investigated.

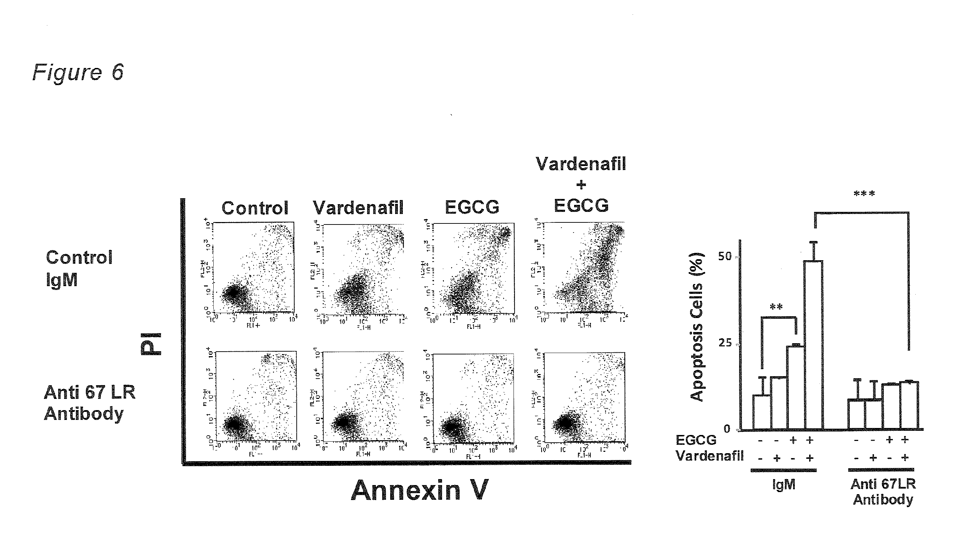

An acute myeloid leukemia cell line, HL60, was seeded in a 96-well plate at 1×10⁴ cells/mL, and vardenafil was added thereto at a final concentration of 5 µM. Immediately after the addition, an anti-67LR monoclonal antibody, MLuc5, was added thereto at a final concentration of 20 µg/mL. In the group of adding an isotype control antibody, mouse IgM was added at a final concentration of 20 µg/mL. After 3 hours, EGCG was added thereto at a final concentration of 5 µM. After 96 hours, apoptotic cell ratio was measured by Annexin V PI double staining. The results are shown in Figure 6 (n=3, Tukey's test, ***: P<0.001, **: P<0.01).

As shown in Figure 6, any cytotoxicity was not observed with vardenafil alone, whereas apoptosis was induced by EGCG alone. Furthermore, the apoptosis-inducing activity of EGCG was notably enhanced by its combined use with vardenafil. The apoptosis-inducing activities by EGCG alone and by the combination of EGCG and vardenafil were completely cancelled by the treatment with the anti-67LR antibody. These results demonstrate that vardenafil notably enhances the 67LR-dependent apoptosis-inducing activity of EGCG on cancer cells.

### (7) Effect of combination of EGCG and PDE5 inhibitor in cell-killing activity (A) and cytostatic activity (B) of EGCG on human prostate cancer cell line PC3

Enhancement of the activity of EGCG on prostate cancer by a selective inhibitor (vardenafil) for PDE5, which is an enzyme specifically degrading cGMP and is believed to be an EGCG resistant factor, was investigated.

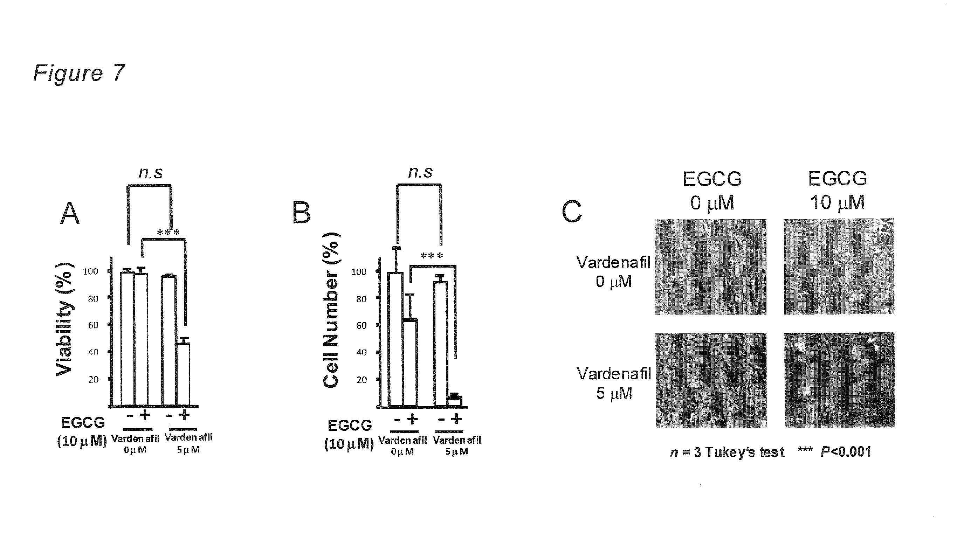

A human prostate cancer cell line, PC3, was seeded in a 24-well plate at 1×10⁴ cells/mL. After pre-culturing for 24 hours, the medium was replaced with a medium containing vardenafil so as to give a final concentration of 5 µM. After 2 hours, EGCG was added thereto at a final concentration of 10 µM. After 96 hours, cell viability determination and viable cell count were performed by trypan blue staining using a hemocytometer. The results are shown in Figure 7 (n=3, Tukey's test, ***: P<0.001).

As shown in Figure 7, though any cytotoxicity was not observed with EGCG and vardenafil alone, a combination of EGCG and vardenafil significantly strongly decreased the viability of cancer cells: (A). The activity of EGCG on viable cell number was notably enhanced by the presence of vardenafil: (B). The same results were obtained also in the microscopic photograph: (C). These results demonstrate that vardenafil, a PDE5-selective inhibitor, notably enhances the activity of EGCG on prostate cancer.

### (8) Effect of combined use of EGCG and PDE5 inhibitor in cell-killing activity and cytostatic activity of EGCG on human multiple myeloma cell line RPMI8226

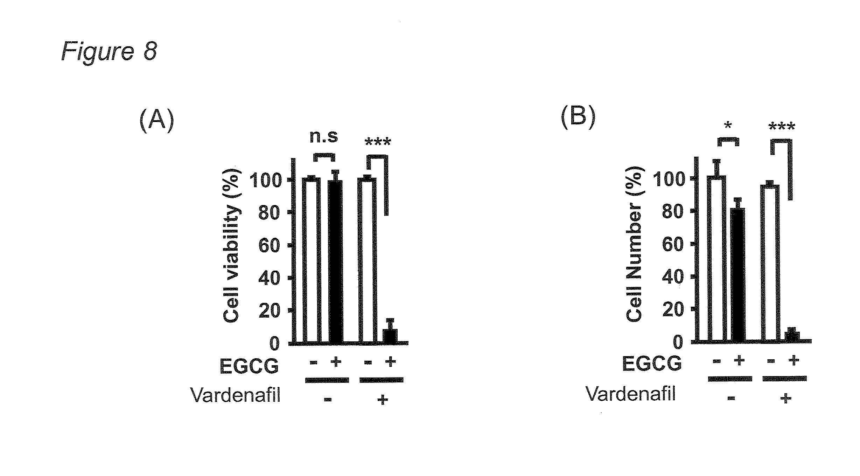

Enhancement of the activity of EGCG on multiple myeloma cells by vardenafil was investigated. A human multiple myeloma cell line, RPMI8226, was seeded in a 24-well plate at 5×10⁴ cells/mL, and vardenafil was added thereto at a final concentration of 5 µM. After 3 hours, EGCG was added thereto at a final concentration of 5 µM. After 96 hours, cell viability (A) and the number (B) of viable cells were measured by trypan blue staining. The value of a control group of cells not treated with EGCG and vardenafil was defined as 100%. The results are shown in Figure 8 (n=3, Tukey's test, *: P<0.05, ***: P<0.001).

As shown in Figure 8, though any cytotoxicity was not observed with EGCG and vardenafil alone, a combination of EGCG and vardenafil significantly strongly decreased the viability of cancer cells: (A). The activity of EGCG on viable cell number was notably enhanced by the presence of vardenafil: (B).

### (9) Effect of combined use of EGCG and PDE5 inhibitor in cell-killing activity and cytostatic activity of EGCG on multiple myeloma derived from patient

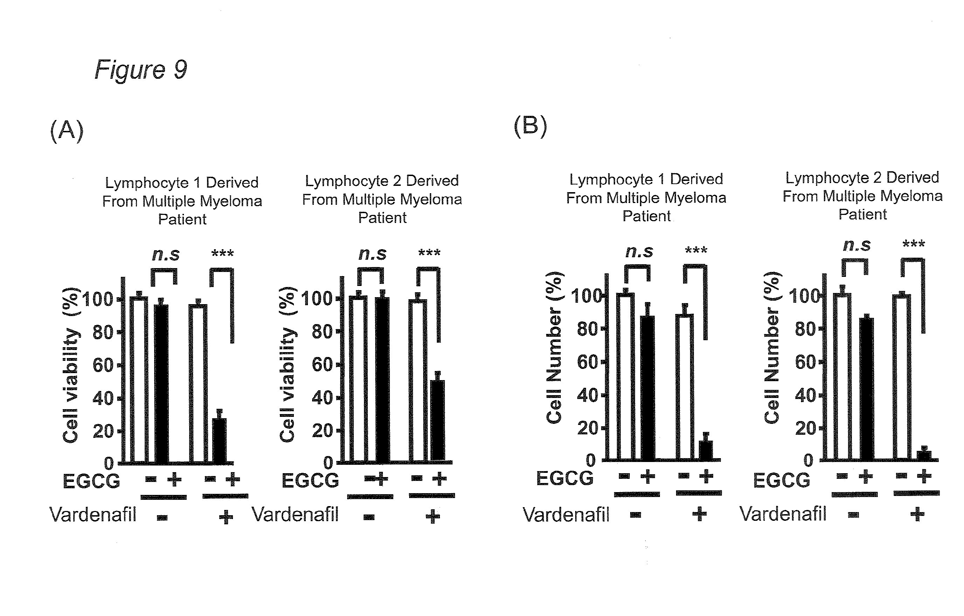

Enhancement of the activity of EGCG on multiple myeloma cells derived from a patient by vardenafil was investigated. Multiple myeloma cells from a patient were seeded in a 24-well plate at 5×10⁴ cells/mL, and vardenafil was added thereto at a final concentration of 5 µM. After 3 hours, EGCG was added thereto at a final concentration of 5 µM. After 96 hours, cell viability (A) and the number (B) of viable cells were measured by trypan blue staining. The value of a control group of cells not treated with EGCG and vardenafil was defined as 100%. The results are shown in Figure 9 (n=3, Tukey's test, ***: P<0.001).

As shown in Figure 9, the combined use of EGCG and vardenafil showed a notable cytostatic activity on the lymphocytes derived from a multiple myeloma patient.

### (10) Effect of combined use of EGCG and PDE5 inhibitor in cell-killing activity and cytostatic activity of EGCG on human plasmacytoma cell ARH77

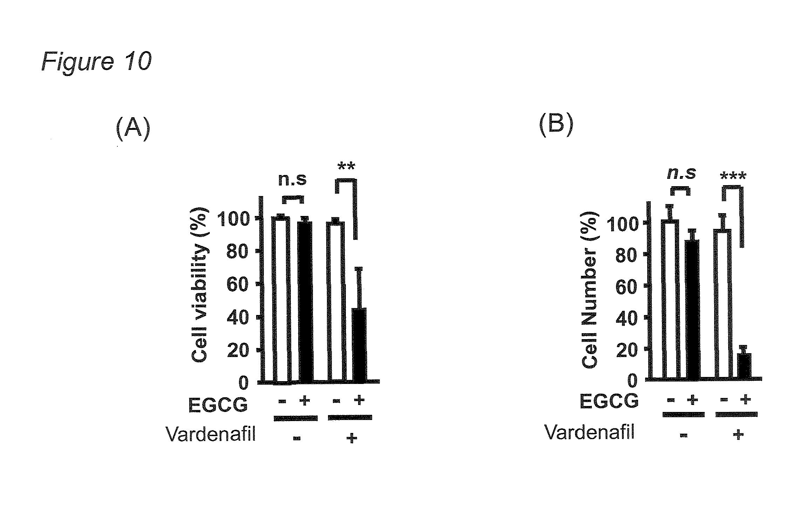

Enhancement of the activity of EGCG on plasmacytoma by vardenafil was investigated. A human plasmacytoma cell, ARH77, was seeded in a 24-well plate at 5×10⁴ cells/mL, and vardenafil was added thereto at a final concentration of 5 µM. After 3 hours, EGCG was added thereto at a final concentration of 5 µM. After 96 hours, cell viability (A) and the number (B) of viable cells were measured by trypan blue staining. The value of a control group of cells not treated with EGCG and vardenafil was defined as 100%. The results are shown in Figure 10 (n=3, Tukey's test, **: P<0.01, ***: P<0.001).

As shown in Figure 10, the combined use of EGCG and vardenafil showed a notable cytostatic activity on the human plasmacytoma cell line.

### (11) Effect of combined use of EGCG and PDE5 inhibitor in cell-killing activity and cytostatic activity of EGCG on chronic myeloid leukemia cell line K562

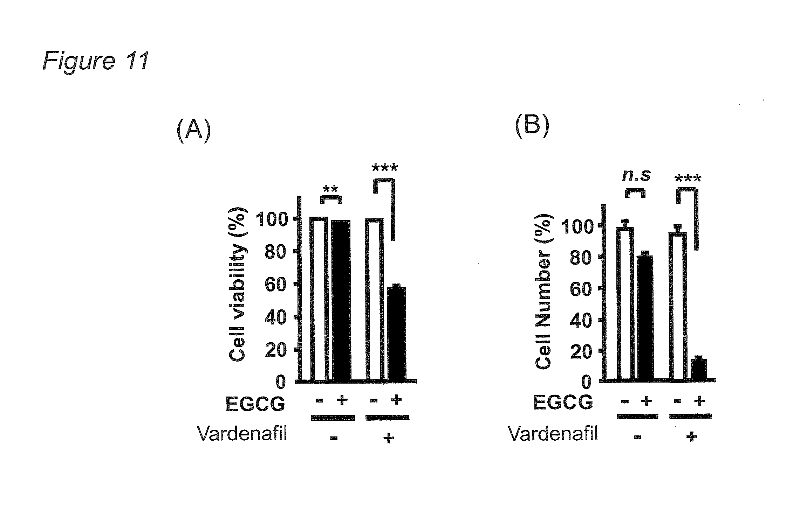

Enhancement of the activity of EGCG on chronic myeloid leukemia cells by vardenafil was investigated. A human chronic myeloid leukemia cell line, K562, was seeded in a 24-well plate at 5×10⁴ cells/mL, and vardenafil was added thereto at a final concentration of 5 µM. After 3 hours, EGCG was added thereto at a final concentration of 5 µM. After 96 hours, cell viability (A) and the number (B) of viable cells were measured by trypan blue staining. The value of a control group of cells not treated with EGCG and vardenafil was defined as 100%. The results are shown in Figure 11 (n=3, Tukey's test, **: P<0.01, ***: P<0.001).

As shown in Figure 11, the combined use of EGCG and vardenafil showed a notable cytostatic activity on the human chronic myeloid leukemia cell line.

### (12) Effect of combined use of EGCG and PDE5 inhibitor in cytostatic activity of EGCG on human breast cancer cell line MCF-7

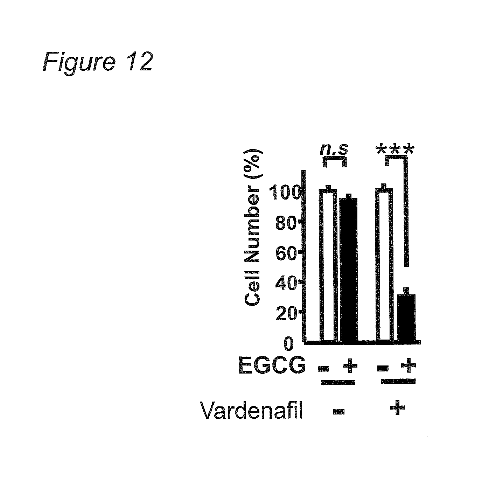

Enhancement of the activity of EGCG on breast cancer cells by vardenafil was investigated. A human breast cancer cell line, MCF-7, was seeded in a 24-well plate at 1×10⁴ cells/mL and was pre-cultured for 24 hours. Subsequently, vardenafil was added thereto at a final concentration of 5 µM. After 3 hours, EGCG was added thereto at a final concentration of 5 µM. After 96 hours, the number of viable cells was measured by trypan blue staining. The value of a control group of cells not treated with EGCG and vardenafil was defined as 100%. The results are shown in Figure 12 (n=3, Tukey's test, ***: P<0.001).

As shown in Figure 12, the combined use of EGCG and vardenafil showed a notable cytostatic activity on the human breast cancer cell line.

### (13) Effect of combined use of EGCG and PDE5 inhibitor in cytostatic activity of EGCG on human gastric cancer cell line MKN45

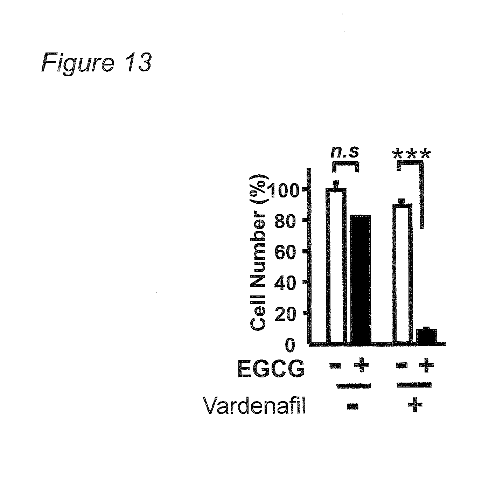

Enhancement of the activity of EGCG on gastric cancer cells by vardenafil was investigated. A human gastric cancer cell line, MKN45, was seeded in a 24-well plate at 1×10⁴ cells/mL and was pre-cultured for 24 hours. Subsequently, vardenafil was added thereto at a final concentration of 5 µM. After 3 hours, EGCG was added thereto at a final concentration of 5 µM. After 96 hours, the number of viable cells was measured by trypan blue staining. The value of a control group of cells not treated with EGCG and vardenafil was defined as 100%. The results are shown in Figure 13 (n=3, Tukey's test, ***: P<0.001).

As shown in Figure 13, the combined use of EGCG and vardenafil showed a notable cytostatic activity on the human gastric cancer cell line.

### (14) Effect of combined use of EGCG and PDE5 inhibitor in cell-killing activity and cytostatic activity of EGCG on human pancreatic cancer cell line PANC-1

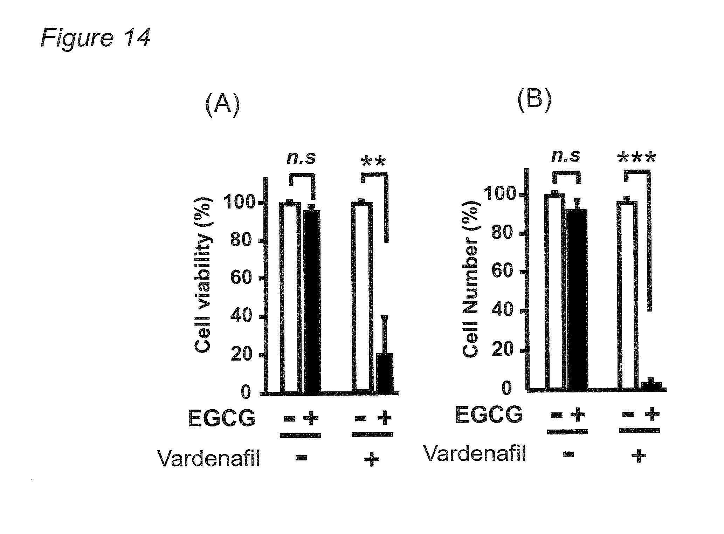

Enhancement of the activity of EGCG on pancreatic cancer cells by vardenafil was investigated. A human pancreatic cancer cell line, PANC-1, was seeded in a 24-well plate at 1×10⁴ cells/mL and was pre-cultured for 24 hours. Subsequently, vardenafil was added thereto at a final concentration of 5 µM. After 3 hours, EGCG was added thereto at a final concentration of 5 µM. After 96 hours, cell viability (A) and the number (B) of viable cells were measured by trypan blue staining. The value of a control group of cells not treated with EGCG and vardenafil was defined as 100%. The results are shown in Figure 14 (n=3, Tukey's test, **: P<0.01, ***: P<0.001).

As shown in Figure 14, the combined use of EGCG and vardenafil showed a notable cytostatic activity on the human pancreatic cancer cell line.

### (15) Effect of combined use of EGCG and PDE5 inhibitor on viability and cell proliferation of human umbilical vein endothelial cell HUVEC

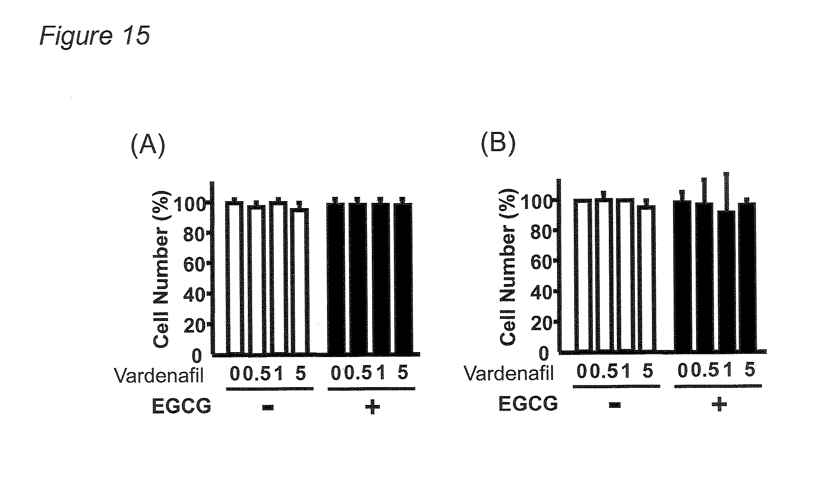

Enhancement of the activity of EGCG on umbilical vein endothelial cells by vardenafil was investigated. A human umbilical vein endothelial cell, HUVEC, was seeded in an EGM-2 medium supplemented with 5% FCS in a 24-well plate at 2×10⁴ cells/mL and was pre-cultured for 24 hours. Subsequently, the medium was replaced with an RPMI 1640 medium supplemented with 1% FCS, SOD (5 U/mL) containing various concentrations (0 to 5 µM) of vardenafil, and Catalase (200 U/mL), followed by treatment for 3 hours. Subsequently, EGCG was added thereto at a final concentration of 5 µM. After 96 hours, cell viability (A) and the number (B) of viable cells were measured by trypan blue staining. The value of a control group of cells not treated with EGCG and vardenafil was defined as 100%. The results are shown in Figure 15 (n=3, Tukey's test).

As shown in Figure 15, the combined use of EGCG and vardenafil did not show any toxicity to the normal cells (HUVEC).

### (16) Effect of combined use of EGCG and PDE5 inhibitor on viability and cell proliferation of peripheral blood lymphocyte derived from healthy subject

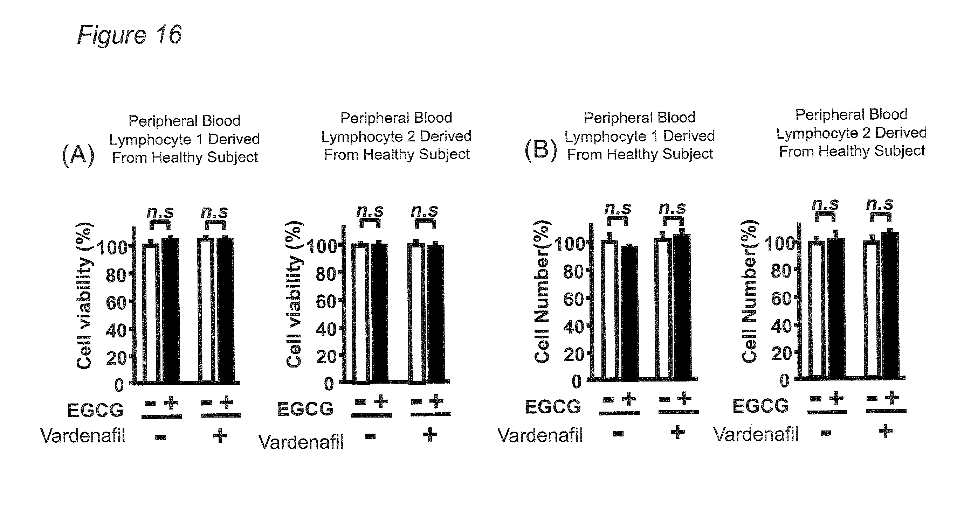

Enhancement of the activity of EGCG on peripheral blood lymphocytes derived from a healthy subject by vardenafil was investigated. Peripheral blood lymphocytes PBMC derived from a healthy subject were seeded in a 24-well plate at 5×10⁴ cells/mL, and vardenafil was added thereto at a final concentration of 5 µM. After 3 hours, EGCG was added thereto at a final concentration of 5 µM. After 96 hours, cell viability (A) and the number (B) of viable cells were measured by trypan blue staining. The value of a control group of cells not treated with EGCG and vardenafil was defined as 100%. The results are shown in Figure 16 (n=3, Tukey's test).

As shown in Figure 16, the combined use of EGCG and vardenafil did not show any toxicity to the normal cells (peripheral blood lymphocytes).

### (17) Effect of combined use of EGCG and PDE5 inhibitor on tumor formation of mouse plasmacytoma cell line MPC11 transplanted to Balb/c mouse

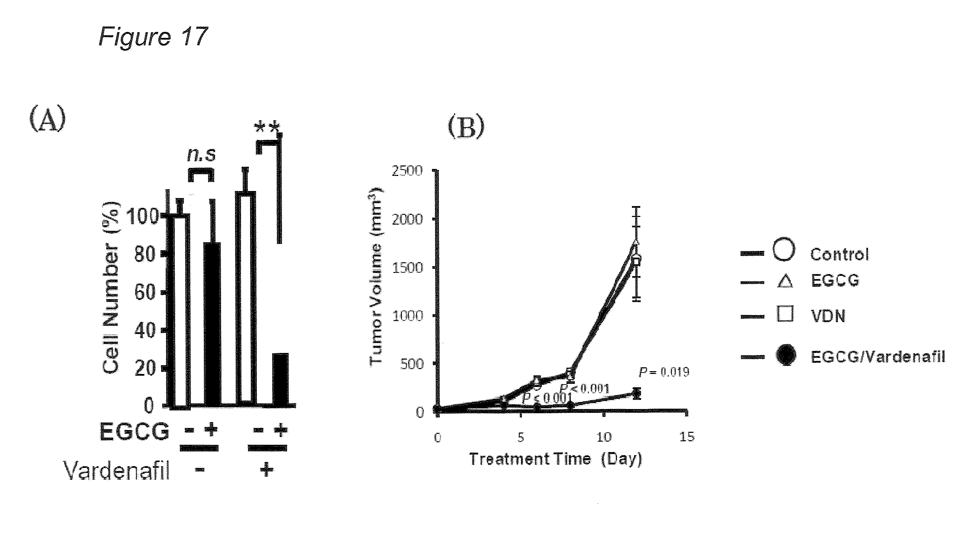

Enhancement of the activity of EGCG on plasmacytoma by vardenafil was investigated. A mouse plasmacytoma cell line, MPC11, was seeded in a 24-well plate at 5×10⁴ cells/mL, and vardenafil was added thereto at a final concentration of 5 µM. After 3 hours, EGCG was added thereto at a final concentration of 5 µM. After 96 hours, the number of viable cells was measured by trypan blue staining. The value of a control group of cells not treated with EGCG and vardenafil was defined as 100%. The results are shown in Figure 17(A) (n=3, Tukey's test, **: P<0.01).

As shown in Figure 17(A), the combined use of EGCG and vardenafil showed a notable cytostatic activity on the mouse plasmacytoma cell line.

Furthermore, 5-week old female balb/c mice were preliminarily bred for one week and were then inoculated with a mouse multiple myeloma cell line, MPC11, by subcutaneous injection, in the right back, of 100 mL of a suspension of 5×10⁷ cells/mL of the cells in an RPMI medium. After 4 days from the inoculation, EGCG (15 mg/kg i.p.) and vardenafil (5 mg/kg i.p.) were administered once every two days, and the tumor volume was measured with varnier clipers. The results are shown in Figure 17(B). In the graph, EGCG and VDN indicate single administration of EGCG and vardenafil, respectively, and EGCG/Vardenafil indicates combination administration of EGCG and vardenafil.

As shown in Figure 17(B), though any inhibitory action on tumor formation of the mouse plasmacytoma cell line was not observed in the groups of administration of EGCG and vardenafil alone, a notable anti-tumor activity was observed in the group of combination administration of EGCG and vardenafil.

### (18) Effect of combined use of methylated EGCG and PDE5 inhibitor on human multiple myeloma cell line U266

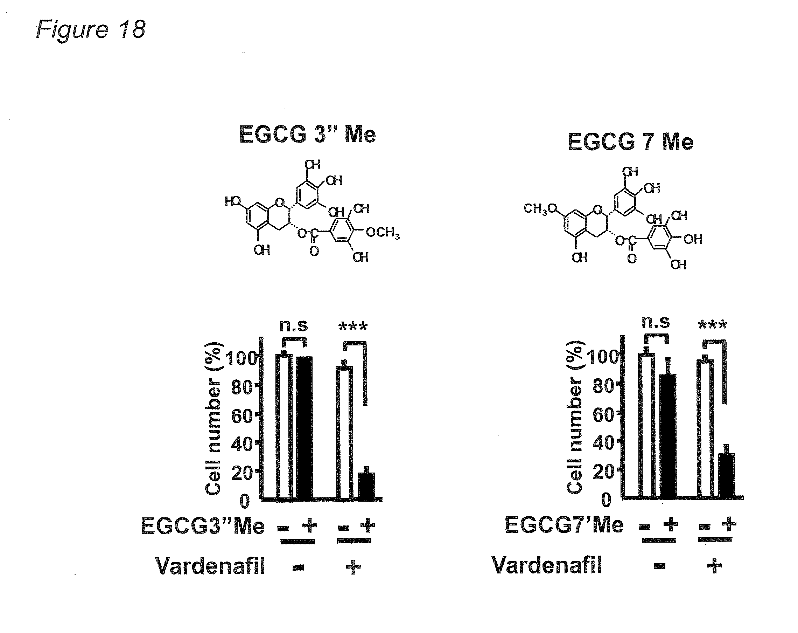

A human multiple myeloma cell line, U266, was seeded in a 24-well plate at 5×10⁴ cells/mL, and vardenafil was added thereto at a final concentration of 5 µM. After 3 hours, EGCG 3" methyl or EGCG 7 methyl was added thereto at a final concentration of 5 µM. After 96 hours, the number of viable cells was measured by trypan blue staining. The value of a control group of untreated cells was defined as 100%. The value of a control group of cells not treated with EGCG 3" methyl, EGCG 7 methyl, and vardenafil was defined as 100%. The results are shown in Figure 18 (n=3, Tukey's test, ***: P<0.001).

As shown in Figure 18, both EGCG 3" methyl and EGCG 7 methyl showed notable cytostatic activities on the multiple myeloma cells by being used in combination with vardenafil.

### (19) Effect of combined use of methylated EGCG and PDE5 inhibitor on prostate cancer cell line PC-3 and pancreatic cancer cell line PANC-1

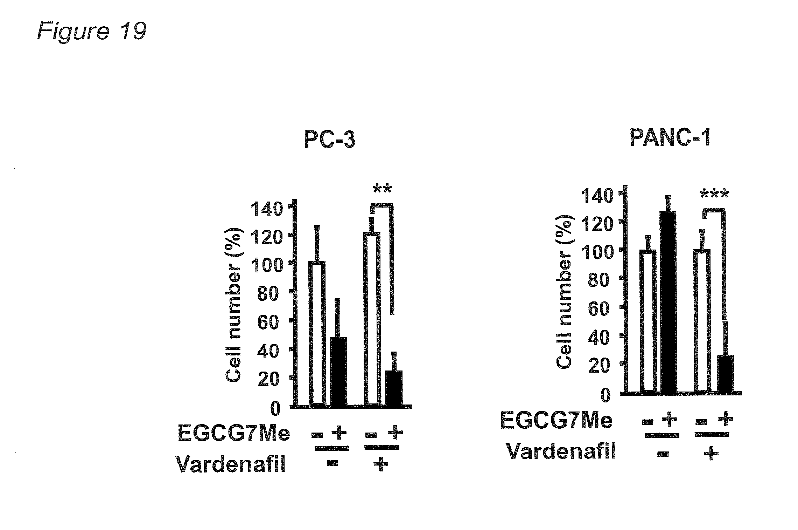

A human prostate cancer cell line, PC3, or a human pancreatic cancer cell line, PANC-1, was seeded in a 24-well plate at 1×10⁴ cells/mL and was pre-cultured for 24 hours. Subsequently, vardenafil was added thereto at a final concentration of 5 µM. After 3 hours, EGCG 7 methyl was added thereto at a final concentration of 5 µM. After 96 hours, the number of viable cells was measured by trypan blue staining. The value of a control group of untreated cells was defined as 100%. The results are shown in Figure 19 (n=3, Tukey's test, **: P<0.01, ***: P<0.001).

As shown in Figure 19, EGCG 7 methyl notably suppressed the cell proliferation of the prostate cancer cell line and the pancreatic cancer cell line by being used in combination with vardenafil.

### (20) Effect of combined use of EGCG and PDE3 inhibitor in cytostatic activity of EGCG on chronic myeloid leukemia cell line K562

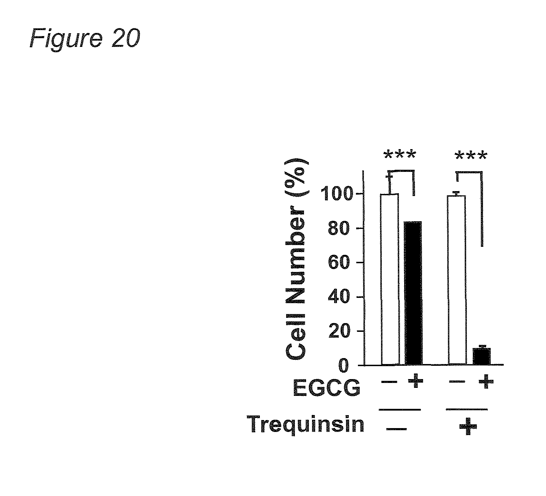

Enhancement of the activity of EGCG on chronic myeloid leukemia cells by a selective inhibitor, trequisin, for phosphodiesterase III was investigated. A human chronic myeloid leukemia cell line, K562, was seeded in a 24-well plate at 5×10⁴ cells/mL, and trequisin was added thereto at a final concentration of 5 µM. After 3 hours, EGCG was added thereto at a final concentration of 5 µM. After 96 hours, the number of viable cells was measured by trypan blue staining. The value of a control group of cells not treated with EGCG and trequisin was defined as 100%. The results are shown in Figure 20 (n=3, Tukey's test, ***: P<0.001).

As shown in Figure 20, EGCG notably suppressed the cell proliferation of the chronic myeloid leukemia cell line by being used in combination with trequisin.

### (21) Enhancement of anti-inflammatory activity (reducing activity on expression of Toll-like receptor (TLR) 2 and TLR 4) of EGCG by its combined use with vardenafil

A mouse macrophage cell line, RAW 264.7, was seeded in a 5-mL dish at 1×10⁵ cells/mL and was pre-cultured in a DMEM medium supplemented with 1% FCS for 24 hours. Subsequently, vardenafil was added thereto at a concentration of 5 µM. After 3 hours, EGCG was added thereto at a concentration of 0 µM or 10 µM. After 24 hours, the cells were collected, and the expressions of Toll-like receptor (TLR) 2 and TLR 4 were measured by Western blotting. The results are shown in Table 1. EGCG notably reduced the expression levels of TLR 2 and TLR 4 by being used in combination with vardenafil.

### [Table 1]

**Table 1**

| | | | | |
|---|---|---|---|---|
| Vardenafil (5 µM) | - | - | + | + |
| EGCG (10 µM) | - | + | - | + |
| TLR2 expression level(%) | 100 | 90 | 112 | 16 |
| TLR4 expression level(%) | 100 | 57 | 112 | 16 |

### [Consideration]

The results described above revealed that a PDE3 inhibitor and a PDE5 inhibitor are effective as enhancers of the anti-cancer activity of EGCG. In addition, it was shown that the combined use of EGCG and a PDE5 inhibitor is considerably effective as a molecular target drug for 67LR-positive cancers. Furthermore, it was revealed that the combination administration of EGCG and a PDE5 inhibitor does not show toxicity to normal cells.

### [0108] [References]

(1) Khan N, Afaq F, Saleem M, Ahmad N, Mukhtar H, et al. Targeting multiple signaling pathways by green tea polyphenol (-)-epigallocatechin-3-gallate.Cancer res, 2006;66:2500-2505.
(2) Shanafelt TD, Call TG, and Zent CS, et al. Phase I trial of daily oral Polyphenon E in patients with asymptomatic Rai stage 0 to II chronic lymphocytic leukemia. J Clin Oncol, 2009;27:3808-3814.
(3) Tachibana H, Koga K, Fujimura Y, Yamada K. A receptor for green tea polyphenol EGCG. Nat Struct Mol Biol, 2004;11:380-381.
(4) Umeda D, Tachibana H, Yamada K. Epigallocatechin-3-O-gallate disrupts stress fibers and the contractile ring by reducing myosin regulatory light chain phosphorylatio mediated through the target molecule 67 kDa laminin receptor. Biochem Biophys Res Commun, 2005;333:628-635.
(5) Umeda D, Yano S, Yamada K, Tachibana H. Involvement of 67-kDa laminin receptor-mediated myosin phosphatase activation in antiproliferative effect of epigallocatechin-3-O-gallate at a physiological concentration on Caco-2 colon cancer cells. Biochem Biophys Res Commun, 2008;371:172-176.
(6) Umeda D, Yano S, Yamada K, Tachibana H. Green tea polyphenol epigallocatechin- 3-gallate (EGCG) signaling pathway through 67-kDa laminin receptor. J Biol Chem, 2008;283:3050-3058.
(7) Nelson J, McFerran NV, Pivato G, et al. The 67 kDa laminin receptor: structure, function and role in disease. Biosci Rep, 2008;28:33-48.
(8) Cioce V, Castronovo V, and Shmookler BM, et al. Increased expression of the laminin receptor in human colon cancer. J Natl Cancer Inst, 1991;83:29-36.
(9) Menard S, Castronovo V, Tagliabue E, Sobel ME. New insights into the metastasis- associated 67 kD laminin receptor. J Cell Biochem, 1997;67:155-165.
(10) Menard S, Taqliabue E, Colnaghi MI. The 67 kDa laminin receptor as a prognostic factor in human cancer. Breast Cancer Res Treat, 1998;52:137-145.
(11) Vacca A, Ribatti D, and Roncali L, et al. Melanocyte tumor progression is associated with changes in angiogenesis and expression of the 67-kilodalton laminin receptor. Cancer, 1993;72:455-461.
(12) Sanjuan X, Fernandez PL, and Miquel, et al. Overexpression of the 67-kD laminin receptor correlates with tumour progression in human colorectal carcinoma. J Pathol, 1996;179:376-380.
(13) Chen FX, Qian YR, Duan YH, et al. Down-regulation of 67LR reduces the migratory activity of human glioma cells in vitro. Brain Res Bull, 2009;79:402-408.
(14) Fontanini G, Vignati S, and Chine S, et al. 67-Kilodalton laminin receptor expression correlates with worse prognostic indicators in non-small cell lung carcinomas. Clin Cancer Res, 1997;3:227-231.
(15) Basolo F, Pollina L, and Pacini F, et al. Expression of the Mr 67,000 laminin receptor is an adverse prognostic indicator in human thyroid cancer: an immunohistochemical study. Clin Cancer Res, 1996;2:1777-1780.
(16) Britschgi A, Simon HU, Tobler A, Fey MF, Tschan MP. Epigallocatechin-3-gallate induces cell death in acute myeloid leukaemia cells and supports all-trans retinoic acid-induced neutrophil differentiation via death-associated protein kinase 2. Br J Haematol, 2010;149:55-64.
(17) Shammas MA, Neri P, Koley H, et al. Specific killing of multiple myeloma cells by (-)-epigallocatechin-3-gallate exracted from green tea: activity and therapeutic implications. Blood, 2006;108:2804-2810.
(18) Keisuke Hirotsu, et al., Apoptosis induction mechanism of EGCG through green tea catechin receptor 67LR, The Sixth Japanese Society of Catechinology, Abstract, p. 41
(19) Rivero-Vilches FJ, de Frutos S, Saura M, Rodriguez-Puyol D, Rodriguez-Puyol M, et al. Differential relaxing responses to particulate or soluble guanylyl cyclase activation on endothelial cells: a mechanism dependent on PKG-I alpha activation by NO/cGMP. Am J Physiol Cell Physiol, 2003;285:C891-898.
(20) Ravipati G, McClung JA, Aronow WS, Peterson SJ, Frishman WH.Type 5 phosphodiesterase inhibitors in the treatment of erectile dysfunction and cardiovascular disease. Cardiol Rev. 2007;15:76-86.
(21) Valiquette L, Montorsi F, Auerbach S. Vardenafil demonstrates first-dose success and reliability of penetration and maintenance of erection in men with erectile dysfunction - RELY-II. Can Urol Assoc J, 2008;2:187-195.
(22) Boolell M, Allen MJ, Ballard SA, Gepi-Attee S, Muirhead GJ, Naylor AM, Osterloh IH, Gingell C, et al. Sildenafil: an orally active type 5 cyclic GMP-specific phosphodiesterase inhibitor for the treatment of penile erectile dysfunction. Int J Impot Res, 1996;8:47-52.
(23) Masaaki Nakahara, et al., Significance of 5'-Nucleotide phosphodiesterase-V as tumor marker in malignant tumor liver metastasis: Comparative review with other tumor markers, The Japanese Journal of Gastroenterological Surgery, The 23rd General Meeting of the Japanese Society of Gastroenterological Surgery, Jpn. J. Gastroenterol. Soc. Vol. 17, No. 2, p. 196

## Claims

1. A pharmaceutical composition comprising a combination of epigallocatechin gallate or a methylated epigallocatechin gallate or a pharmaceutically acceptable salt thereof and a phosphodiesterase inhibitor.

2. The composition according to claim 1, which composition is used in treatment of cancer, allergy, obesity, arteriosclerosis, inflammation, or muscle atrophy.

3. The composition according to claim 1 or 2, which composition is used in treatment of a 67LR-positive disease.

4. The composition according to any one of claims 1 to 3, wherein the phosphodiesterase inhibitor is a phosphodiesterase 3 inhibitor or phosphodiesterase 5 inhibitor.

5. The composition according to any one of claims 1 to 3, wherein the phosphodiesterase inhibitor is vardenafil.

6. A kit for use in treatment of cancer, allergy, obesity, arteriosclerosis, inflammation, or muscle atrophy, the kit comprising:
a) a first unit dosage form containing epigallocatechin gallate, a methylated epigallocatechin gallate, or a pharmaceutically acceptable salt thereof;
b) a second unit dosage form containing a phosphodiesterase inhibitor; and
c) a container device for containing the first unit dosage form and the second unit dosage form.
